# EUROPÄISCHE PATENTSCHRIFT

(11) **EP 3 131 409 B1**
(45) Veröffentlichungstag und Bekanntmachung des Hinweises auf die Patenterteilung: **25.06.2025**
(21) Anmeldenummer: 15709169.5
(22) Anmeldetag: 11.03.2015
(51) Int. Cl.: A23K 10/00, A23K 40/25, A61P 43/00, A23K 50/10, A23K 20/158, A23K 40/10, A61K 31/20, A61K 45/06, A23C 9/14, A23C 9/00, A23K 10/40, A23K 50/00, A61K 31/201, A61K 31/202, A23K 40/20

(54) **PELLETIERTES WIEDERKÄUERFUTTER ANGEREICHERT MIT PANSEN-LABILEN INHALTSSTOFFEN**
PELLETED RUMINANT FEED ENRICHED WITH RUMEN-AVAILABLE INGREDIENTS
ALIMENT POUR RUMINANTS EN GRANULÉS ENRICHI AVEC DES INGRÉDIENTS INSTABLES DANS LA PANSE

(30) Priorität: 13.03.2014 EP 14159496
(43) Veröffentlichungstag der Anmeldung: 22.02.2017
(73) Patentinhaber: BASF SE, 67056 Ludwigshafen am Rhein (DE)
(72) Erfinder: TRÖSCHER, Arnulf, 69469 Weinheim (DE); OBERFRANK, Uwe, 67354 Römerberg (DE)
(74) Vertreter: BASF IP Association
(86) Internationale Anmeldenummer: PCT/EP2015/055006
(87) Internationale Veröffentlichungsnummer: WO 2015/135961

(56) Entgegenhaltungen:
- EP-A1- 2 676 551
- WO-A1-2013/113981
- WO-A1-99/66922
- WO-A2-2007/149818
- DE-A1- 102004 013 464
- US-A1- 2006 045 957

## Beschreibung

Die Erfindung betrifft pelletiertes Wiederkäuer-Futtermittel, enthaltend in pelletierter Form eine Mischung wenigstens eines festen partikelförmigen Futtermittelbestandteils mit wenigstens einem der Mischung zugesetzten Pansen-instabilen Inhaltsstoff; Verfahren zu deren Herstellung und deren Anwendung in Verfahren zur Änderung der Konzentration von Milchfett.

### Hintergrund der Erfindung

Zusätze zu Wiederkäuerfutter können unerwünschten chemischen Veränderungen durch Pansenorganismen unterliegen. So ist im Rahmen der Modifizierung von mehrfach ungesättigten Fettsäuren durch die Pansenmikroben zu beobachten, dass sich sowohl der Gehalt als auch die Lage von Doppelbindungen in der Kohlenstoffkette der Fettsäure verändern kann. Üblicherweise sind die Doppelbindungen der ungesättigten Fettsäuren in den Pflanzen durch mindestens zwei Einfachbindungen getrennt .Mikrobielle Enzyme können ein konjugiertes Doppelbindungssystem generieren, bei dem die Doppelbindungen nur durch eine Einfachbindung getrennt sind. Hierzu gehören beispielsweise die konjugierten Linolsäure-Isomere (conjugated linoleic acids, CLA). Sie entstehen vor allem durch die Linolsäure-Isomerase des Pansenbakteriums *Butyrivibrio fibrisolvens* aus Linolsäure (C18:2 cis-9, trans-12). Das Haupt-CLA-Isomer ist die cis-9, trans-11-CLA (C18:2 cis-9, trans-11) mit etwa 75-92 % am Gesamt-CLA-Gehalt. Demgegenüber ist das Milchfett-depressiv wirkende Isomer C18:2 trans-10, cis-12 nur mit einem geringen Anteil von 0,03- 1,5 % vertreten. Von diesen Isomeren gelangt ein Teil über die Absorption in den Organismus des Wiederkäuers. Bei Milchkühen werden die pansenmodifizierten Fettsäuren auch in die Milch transportiert. Der CLA-Gehalt des Milchfettes ist stark fütterungsabhängig und liegt bei etwa 0,3-1,1%. (vgl. Kirchgeßner, Tierernährung, 13. Auflage, DLG Verlag GmbH), bei Weidefütterung auch bis zu über 4% (Tröscher et al., 2014; im Druck)
CLAs werden aufgrund ihrer starken physiologischen bzw. pharmakologischen Wirkungen intensiv beforscht. In Zellkulturstudien und Tierversuchen zeigen CLAs krebsprotektive und antientzündliche Wirkungen. Es ist auch bekannt, dass konjugierte Linolsäure-Isomere (CLA-Isomere) bei Wiederkäuern die Expression zahlreicher Gene hemmen, die für die Aufnahme von zirkulierenden Fettsäuren in die Milchdrüse, für die Synthese von Fettsäuren sowie die Triglyceridbildung verantwortlich sind. Hierbei hat sich vor allem das C18:2 trans-10, cis-12 CLA-Isomer als besonders fettreduzierend erwiesen. Im Zusammenhang mit der Abnahme des Fettanteils in der Milch werden als Vorteile u,a, die Entlastung des Stoffwechsels sowie Erhöhung der Milchmenge diskutiert. So wird berichtet, dass Senkungen des Milchfettgehaltes von 0,4-0,5 % zu Steigerungen der Milchmenge um bis zu 3- 10% führen können. (vgl. Kirchgeßner, a.a.O)

Aus diesem Grund wird heute dem Milchleistungsfutter oftmals CLAs zugesetzt. Diese Präparationen sind in der Fütterung zugelassen, müssen allerdings in pansengeschützter Form eingesetzt werden da ungeschützte CLAs im Pansen weiter zu unwirksamen C18:1 und C18:0 Fettsäuren abgebaut werden.

Wenn diese oder andere Moleküle, die durch Pansen-Mikroorganismen abgebaut oder chemisch so verändert werden, dass sie ihre biochemische Wirkung verlieren, Wiederkäuer verabreicht werden sollen, müssen sie also gegen derart unerwünschte mikrobielle Wirkungen im Pansen geschützt werden. Verschiedene Verfahren sind in der Literatur beschrieben, die mehrfach ungesättigten Fettsäuren (PUFAs), wie den CLAs Pansenschutz verleihen können. Dies verdeutlichen auch Befunden, beispielsweise von Elgersma et al., in Fresh Herbage for Dairy Cattle, 175-194,2006 die Biohydrogenierungsraten von ungesättigten Fettsäuren im Bereich von 82 - 98 % beschreiben. Lediglich etwa 2 bis 22 % der aufgenommenen mehrfach ungesättigten Fettsäuren (PUFAs) sind nach dem Durchgang durch den Pansen noch nachweisbar.

So werden in der Literatur (vgl. Kirchgeßner, a.a.O) verschieden Formen pansengeschützter Fette, die gegen mikrobiellen Abbau und mikrobielle Modifikation geschützt sind, beschrieben:
Erwähnt werden dort insbesondere:
a) nativ geschützte zellgebundene Fette der Ölsaaten, die in Form von Ölsaatkuchen oder geschroteten Vollsaaten eingesetzt werden. Das Schutzprinzip besteht dabei in der langsamen Freisetzung der Öle aus den Pflanzenzellen; allerdings weisen solche Fette eine geringe Lagerstabilitat auf.
b) hitzebehandelte Ölsaaten, z. B. durch Extrusion; wobei denaturierte Eiweiße das Fetttröpfchen umgeben und es so vor mikrobiellem Abbau schützen.
c) chemisch modifizierte Fette, z. B. durch Verseifung von Fettsäuren mit Calcium oder Ummantelung ("Coating) der Fetttröpfchen mit formaldehydbehandelten Proteinen.
d) Fette, die durch technische Verfahren (z. B. Ummantelung mit gehärteten Pflanzenfette) vor mikrobiellen Enzymen geschützt sind.

Aus der EP-A-1 100 489 ist ein Verfahren zur Verringerung des Milchfettgehalts bei Milchvieh bekannt, wobei eine wirksame Menge an CLA dem Vieh zu verabreichen ist. Zum Schutz vor pansenbakterielle Veränderung wird vorgeschlagen den Wirkstoff durch Injektion zu verabreichen oder diesen in gecoateter Form bereit zu stellen.

### Figurenbeschreibung

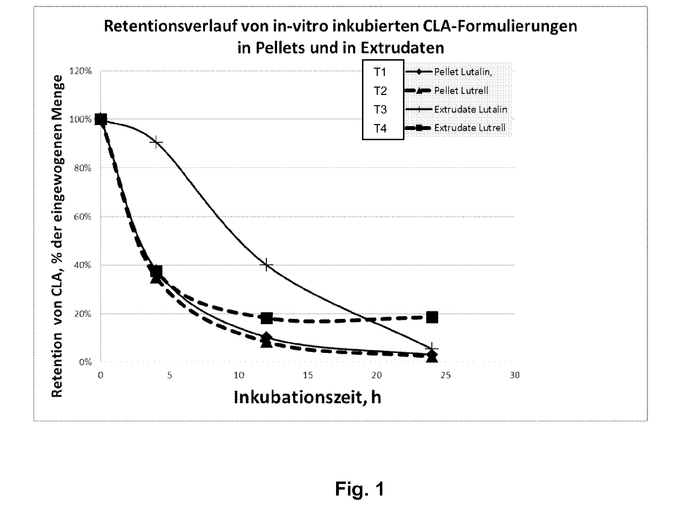

Figur 1 zeigt den zeitlichen Retentionsverlauf von verschiedenen in-vitro inkubierten CLA-Formulierungen in Pellets und Extrudaten (T1 und T3 erfindungsgemäß)

### Zusammenfassung der Erfindung

Aufgabe der Erfindung ist die Bereitstellung eines vereinfachten Wegs zur Überführung Pansen-instabiler Futterinhaltsstoffe in eine Darreichungsform mit verbesserter Pansen-Stabilität.

Überraschenderweise konnte die Aufgabe durch Bereitstellung von ungecoateten, pelletierten Futtermitteln gemäß den beiliegenden Ansprüchen gelöst werden.

### Spezielle Ausgestaltungen der Erfindung

### a) Allgemeine Definitionen

Ein "Pellet" oder eine "pelletiere Form" umfasst feste Futterformulierungen, wie sie mit Hilfe herkömmlicher Pelletiervorrichtungen aber auch herkömmlicher Extrusions- oder Expandiervorrichtungen in an sich bekannter Weise zugänglich sind. Üblicherweise besitzen derartige Pellets eine Länge von etwa 0,5 bis 2 oder 0,8 bis 1,5 cm, wie z.B. etwa 1cm und eine Durchmesser im Bereich von 0,1 bis 0,8 oder 0,4 bis 0, 5 cm. Mitumfasst durch diese Begriffe sind auch sogenannte "gekrümelten" Pellets, d.h. Pellets die durch mechanische Krafteinwirkung in kleinere Partikel überführt werden Zweck des Krümelns ist häufig eine Erleichterung der Aufnahme der Pellets durch das Tier oder auch eine bessere Verteilung im Endfutter. Durch das Krümeln kann man die Partikelgröße gezielt verringern und die Partikelzahl erhöhen. Man erhält dabei z.B. ein Partikelgemisch mit eine Anteil von >50%, z.B. 55 bis 95 oder 60 bis 90 oder 70 bis 80% an Partikeln mit einem Durchmesser im Bereich von etwa 3 bis 6 oder 4 bis 5 mm.

Ein "Pansen-instabiler Inhaltsstoff" (Reinsubstanzen aber auch natürliche oder synthetische Stoffgemische), umfasst eine chemische Verbindung, welche ohne zureichenden Schutz, bei Passage des Wiederkäuerpansens chemischen Veränderungen (Veränderung der chemischen Summenformel, wie z.B. durch Biohydrogenierung, oder und/ oder der Konfiguration und/oder Stereochemie) unterzogen werden kann. In der Regel ist dies eine organisch-chemische Substanz, insbesondere eine solche, welche als Nahrungs-/Futterbestanteil oder Nahrungs/Futterzusatz von Bedeutung ist. Insbesondere sind zu nennen ein- oder mehrfach ungesättigte Carbonsäuren, wie z.B. solche mit mindestens 6 Kohlenstoffatomen, wie z.B. die unten angegebenen CS, PUFA, MUFA oder CLA. Weitere Futteradditive bzw. Futterbestandteile oder Einzelfuttermittel, die Pansen-geschützt verabreicht werden müssen/können sind Aminosäuren (insbesondere. Methionin, Lysin, etc.), Enzyme, Cholin, und andere Wirkstoffe aus der Klasse der Vitamine.

Eine "Pansen-instabile Mischung" oder "Pansen-instabile Futtermittel-Mischung" umfasst wenigstens einen "Pansen-instabilen" Inhaltsstoff, und ist nicht oder unzureichend Pansen geschützt, d.h. der Inhaltsstoff wäre bei Verfütterung und Passage des Wiederkäuerpansens chemischen Veränderungen wie oben beschrieben ausgesetzt.

"Pansen-Stabilität" oder "Pansen-Schutz" im Kontext der Erfindung bedeutet, dass ein "Pansen-instabiler" Inhaltsstoff durch die oben beschriebene "Pellet"-Form in einen Zustand verringerter "Pansen-Instabilität" bis hin zum im Wesentlichen vollständigen Pansenschutz überführt wird. Diese verbesserte Pansen-Stabilität oder verringerte Pansen-Instabilität kann in einfacher Weise durch Vergleich der Stabilität des Inhaltsstoffs in einer pelletierten oder nicht-pelletierten Mischung unter Anwendung von im Experimentellen Teil beschriebenen Versuchsansätzen (in vitro oder in-vivo) bestimmt werden.

"Carbonsäuren" (CS) sind insbesondere geradkettige oder verzweigte, insbesondere geradkettige, gesättigte oder, ein- oder mehrfach ungesättigten, gegebenenfalls substituierten C₆-C₃₀-Monocarbonsäuren Beispiele für gesättigte unverzweigte Fettsäuren sind Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Beispiele für einfach ungesättigte Fettsäuren sind Palmitoleinsäure, Ölsäure und Erucasäure. Beispiele für zweifach ungesättigte Fettsäuren sind Sorbinsäure und Linolsäure. Beispiele für dreifach ungesättigte Fettsäure sind Linolensäure und Elaeostearinsäure. Beispiele für vier- und mehrfach ungesättigte Fettsäuren sind Arachidonsäure, Clupanodonsäure, Eicosapentaensäure und Docosahexaensäure. Beispiele für substituierte Fettsäuren sind Ricinolsäure ((R)-12-Hydroxy-(Z)-9-octadecensäure). Weitere geeignete Fettsäuren sind natürlich vorkommende Fettsäuren wie Gondosäure und Neronsäure. Sind in den Fettsäuren Doppelbindungen enthalten, so können diese sowohl in cis- als auch in transForm vorliegen. Die Substituenten sind vorzugsweise ausgewählt unter Hydroxy- und Niedrigalkylgruppen, wie z.B. Methyl- und Ethylgruppen. Weiterhin können Ketogruppen oder Epoxygruppen, wie z.B. in der Vernolsäure im Kohlenwasserstoffrest enthalten sein. Weitere funktionelle Gruppen sind Cyclopropan-, Cyclopropen- und Cyclopentenringe, welche durch Verbrückung von zwei benachbarten Kohlenstoffatomen im Kohlenwasserstoffrest der Fettsäure ausgebildet werden können (vgl. Malvaliasäure und Chaulmoograsäure).

"PUFA" sind mehrfach ungesättigte Fettsäuren mit mindestens 2 konjugierten oder nicht-konjugierten C=C-Doppelbindungen im Fettsäuremolekül. Als Beispiele sind zu nennen: Linolensäure, Eicosapentaensäure (EPA) ((5*Z*,8*Z*,11*Z*,14*Z*,17*Z*)-Eicosa-5,8,11,14,17-pentaensäure; oder C20:5 (ω-3) ) und Docosahexaensäure (DHA) ((4*Z*,7*Z*,10*Z*,13*Z*,16*Z*,19*Z*)-Docosa-4,7,10,13,16,19-hexaensäure) oder C22:6 (ω-3)).

"MUFA" sind einfach ungesättigte Fettsäuren, die in cis- oder trans-Konfiguration vorkommen wie z.B. die Ölsäure oder die Vaccensäure.

Konjugierte Linolsäuren (CLA) sind eine Gruppe von Isomeren der zweifach ungesättigten C₁₈-Monocarbonsäure "Linolsäure", deren beiden Doppelbindungen in Position 9 und 12 und somit nicht konjugiert vorliegen. Diese wird auch mit der Kurzform "C18:2 cis-9, cis-12" bezeichnet.

"CLA" umfasst grundsätzlich alle konjugierten, zweifach-ungesättigten Isomere der Linolsäure (C18:2 cis-9,cis-12), wobei die Position der beiden Doppelbindungen in der Kohlenstoffkette zum Kettenende oder zur Carboxylgruppe hin verschoben sein kann und außerdem die Stereochemie der konjugierten Doppelbindungen jegliche Variation umfassen kann (cis/cis, trans/trans, cis/trans), wobei "cis/trans" beide Reihenfolgen "trans-cis" oder "cis-trans" umfasst, wobei jeweils die erstgenannte Konfiguration der beiden Reihenfolge die der Carboxylgruppe am nächsten liegende Doppelbindung betrifft:

Beispielhaft sei auf die im Folgenden dargestellte konjugierte Linolsäure "C18:2 cis-9, trans-11" verwiesen:

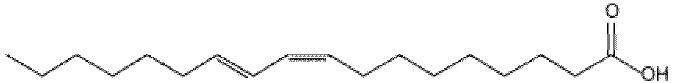

Die hierin verwendeten Bezeichnungen : cis/trans-9,11-Linolsäure; cis/trans-8,10-Linolsäure; cis/trans-11,13-Linolsäure; und cis/trans-10,12-Linolsäure; umfassen somit sowohl die cis-trans als auch die trans-cis Isomere ,also:
cis/trans-9,11-Linolsäure umfasst : C18 :2 cis-9, trans-11 und C18 :2 trans-9,cis 11
cis/trans-8,10-Linolsäure umfasst : C18 :2 cis-8, trans-10 und C18 :2 trans-8,cis 10
cis/trans-11,13-Linolsäure umfasst : C18 :2 cis-11, trans-13 und C18 :2 trans-11,cis 13
cis/trans-10,12-Linolsäure umfasst : C18 :2 cis-10, trans-12 und C18 :2 trans-10,cis 12

Analoges gilt für dreifach ungesättigte Linolensäure (C18 :3, cis-9, cis-12, cis-15) und den cis/trans Isomeren davon.

Obige Stoffangaben und die hierin beschriebenen Verwendungen dieser Stoffe betreffen primär den jeweiligen Reinstoff aber auch natürliche oder synthetische Stoffgemische, welche wenigstens einen dieser Stoffe, z.B. wenigstens ein PUFA oder wenigstens ein CLA enthalten.

Natürliche Stoffgemische sind z.B. Fischöle oder mikrobielle Öle, welche reich an PUFAs sein können, sowie Leinöl, Sojaöl, Sonnenblumenöl, Rizinusöl, etc.

Synthetische Stoffgemische sind beispielsweise CLA -reiche Handelsprodukte wie Lutalin^{®} der BASF SE.

Weiterhin sind zu nennen, Pansen-instabile Derivate der oben genannten Carbonsäuren (PUFA, MUFA, CLA), wie insbesondere substituierte Derivate. Beispielsweise sind zu nennen

Verbindungen die am Kohlenwasserstoffrest der Carbonsäure ein- oder mehrfach substituiert sind, wie z.B. durch Hydroxylgruppen. Als Beispiel für eine derartige Verbindung ist zu nennen: 10-Hydroxy-cis-12-octadecadiensäure.

### b) Besondere Ausführungsformen

Die vorliegende Erfindung ist im beigefügten Anspruchssatz beschrieben.

Die Erfindung betrifft also ein pelletiertes Wiederkäuer-Futtermittel für Milchvieh, enthaltend in ungecoateter, pelletierter Form (d.h. durch Pelletieren, Extrusion, Expansion aber auch in gekrümelter Form, d.h. durch anschließendes Krümeln von Pellets, Extrudaten oder Expandaten erhaltenen kleinere Partikel) eine (Pansen-instabile) Mischung wenigstens eines festen partikelförmigen Futtermittelbestandteils mit wenigstens einem der Mischung zugesetzten Pansen-instabilen Inhaltsstoff, ausgewählt unter wenigstens einer konjugierten Linolsäure (CLA) in Reinform oder als Bestandteil eines natürlichen oder synthetischen Stoffgemischs, wobei insbesondere einzelne, mehrere oder alle Mischungsbestandteile, die zur Pelletierung mit dem Pansen-instabilen Inhaltsstoff vermischt werden sollen, an sich keine oder keine ausreichende Pansen-stabilisierende Wirkung in der zu pelletierenden Mischung zeigen. Bei CLA-haltigen Mischungen sind solche Mischungsbestandteile nicht oder nur in unwirksamer Menge enthalten, die in nichtpelletierten CLA-Mischungen eine Pansenstabilisierung der CLA bewirken würden. Beispielsweise sind hierzu die im Pansen-stabilisierten CLA-Handelsprodukt Lutrell ^{®} enthaltenen stabilisierend wirkenden Zusätze, wie insbesondere Sojaöl, Gips oder Silica zu nennen, die in erfindungsgemäßen, zu pelletierenden Futtermittelmischung nicht oder nur in nicht-stabilisierenden Mengen enthalten sind. Der Anteil an zugesetztem Pansen-instabilen Inhaltsstoff liegt erfindungsgemäß im Bereich von 0,1 bis 20, 1 bis 15, 2 bis 10 oder 3 bis 5 Gew.-%, bezogen auf das Gesamtgewicht der zu pelletierenden Mischung beträgt. Das erfindungsgemäße pelletierte Wiederkäuerfutter wird dadurch hergestellt, dass man
a. wenigstens einen festen partikelförmigen Futtermittelbestandteil mit dem Inhaltsstoff vermischt,
b. die so erhaltene Mischung gegebenenfalls durch Einleiten von bis zu 95°C heißem Wasserdampf konditioniert,
c. die gegebenenfalls konditionierte Mischung in einer Pelletier- Extrudier- oder Expandiervorrichtung unter Einwirkung von Druck und gegebenenfalls Dampf bei Temperaturen im Bereich von 60 bis 100°C zu Pellets verpresst, und
d. die so erhaltenen Pellets gegebenenfalls abkühlt und/oder nachtrocknet und gegebenenfalls anschließend krümelt.

Die zur Herstellung der erfindungsgemäßen Futtermittelpellets eingesetzte Mischung kann daher als "Pansen-instabile" Mischung bezeichnet werden, die erst durch den erfindungsgemäßen Pelletierungs-, Extrusions- oder Expansionsschritt "Pansen-Stabilität" erhält, d.h. deren Pansen-Instabilität dadurch verringert wird.

Daraus folgt, dass erfindungsgemäß keine Futtermittelmischungen zu Pellets verarbeitet werden, die bereits vor der Pelletierung eine ausreichende Pansen-Stabilität besitzen würden. Beispielsweise umfasst daher die Erfindung nicht die Verarbeitung von Pansen-geschützten Inhaltsstoffmischungen (wie z.B. der CLA-Mischung Lutrell ^{®} oder Mischungen vergleichbarer Zusammensetzung) zu pelletiertem Wiederkäuer-Futtermittel.

Erfindungsgemäß ist die CLA ausgewählt unter
a) cis/trans-9,11-Linolsäure,
   d.h. C18:2 cis-9, trans-11 und C18 :2 trans-9,cis 11
b) cis/trans-8,10-Linolsäure;
   d.h.C18 :2 cis-8, trans-10 und C18 :2 trans-8,cis 10
c) cis/trans-11,13-Linolsäure;
   d.h. C18 :2 cis-11, trans-13 und C18 :2 trans-11,cis 13
d) cis/trans-10,12-Linolsäure;
   d.h. C18 :2 cis-10, trans-12 und C18 :2 trans-10,cis 12
e) cis/cis-9,11-Linolsäure,
f) trans/trans-9,11-Linolsäure,
g) cis/cis-8,10-Linolsäure
h) trans/trans-8,10-Linolsäure
i) cis/cis-11,13-Linolsäure
j) trans/trans-11,13-Linolsäure
k) cis/cis-10,12-Linolsäure
l) trans/trans-10,12-Linolsäure; und
m) Gemischen von wenigstens zwei der oben genannten Verbindungen.

Insbesondere ist die CLA ausgewählt unter
a) 9-cis,11-trans- Linolsäure (C18 :2 cis-9, trans-11) ;
b) 10-trans,12-cis-Linolsäure (C18 :2 trans-10,cis 12); und
c) Gemischen davon.

Gegenstand der Erfindung ist auch ein Verfahren zur Herstellung eines erfindungsgemäßen pelletierten Wiederkäuer-Futtermittels wobei man
a) wenigstens einen festen partikelförmigen Futtermittelbestandteil mit wenigstens einem Pansen-instabilen Inhaltsstoff gemäß obiger Definition vermischt,
b) die so erhaltene Mischung gegebenenfalls konditioniert, insbesondere durch Dampfeinleitung, insbesondere Konditionierung mit bis zu 95 °C heißem Wasserdampf von , wie z.B. Sattdampf, über einen ausreichenden Zeitraum, wie z.B. für 20 bis 40 oder 20 bis 120 oder 20 bis 140 oder 20 bis 240 Sekunden,
c) die gegebenenfalls konditionierte Mischung in einer Extrusions-, Expansions- oder einer Pelletiervorrichtung, insbesondere einer Pelletier- oder Extrusionsvorrichtung, unter Einwirkung von Druck und gegebenenfalls Dampf bei einer Temperatur von 60 bis 100°C zu Pellets verpresst; und
d) die so erhaltenen Pellets gegebenenfalls abkühlt und/oder nachtrocknet und gegebenenfalls anschließend krümelt.

Dabei weist die erhaltene pelletierte Mischung im Vergleich zur nicht-pelletierten Mischung eine verbesserte Pansen-Stabilität des Inhaltsstoffs (d.h. eine verringerte Pansen-Instabilität des Inhaltsstoffs bis hin zum vorzugsweise im Wesentlichen vollständigen Pansenschutz) auf.

Weiterhin ist hierin ein Verfahren zur Änderung der Konzentration von Milchfett in Milch beschrieben, was aber nicht Gegenstand der beanspruchten Erfindung ist, die von einem milchspendenden Wiederkäuer erzeugt wird, wobei man dem milchspendenden Wiederkäuer eine wirksame Menge eines wie oben beschriebenen pelletierten Wiederkäuer-Futtermittels verabreicht. Der milchspendende Wiederkäuer ist dabei ausgewählt unter Kuh, Ziege und Schaf.

### d) Weitere Ausgestaltungen

### d1) Herstellung der Futtermittelpellets

Zur Herstellung der pelletierten Futtermittelzusammensetzungen werden der oder die erfindungsgemäß zu formulierenden Pansen-instabilen Inhaltsstoffe ggf. zusammen mit weiteren Zusätzen mit üblichem Tierfutterbestandteilen (wie z. B. Milchleistungsfutter) vermischt. Der Inhaltsstoffanteil wird so gewählt, dass der z. B. im Bereich von 0,1 bis 20, 1 bis 15, 2 bis 10 oder 3 bis 5 Gew.-% liegt. Anschließend wird das Futter mit Hilfe einer geeigneten Pelletpresse pelletiert. Dazu wird das Futtergemisch üblicherweise durch Dampfeinleitung (mit bis zu 95 °C heißem Wasserdampf, z.B. Sattdampf), z.B. für 20 bis 1240 Sekunden konditioniert. Über die zugesetzte Menge an Sattdampf kann die Temperaturerhöhung während des Pelletiervorgangs beeinflusst werden. Diese Dampfkonditionierung kann aber auch entfallen.

Üblicherweise verwendet man zur Pelletierung handelsübliche Pelletpressen (z.B. Fa Bühler GmbH) die als Ringmatrizenpresse ausgestaltet sein können. Das vorkonditionierte Stoffgemisch wird dabei mittels walzen durch eine Ringmatrize (Spaltbreite zwischen Walze und Matrize z.B. 1 mm) gepresst. Je nach Matrize können so Pellets von etwa 2 bis 12 mm Durchmesser hergestellt werden. Für Milchviehfutter verwendet man z.B. Matrizen mit einem Bohrungsdurchmesser von 4 bis 5 mm und einer Lochlänge von 40 bis 50 mm. Die höchste Prozesstemperatur tritt dabei beim Pressen des Gemisches durch die Matrize auf. Hier können Temperaturen im Bereich von etwa 60 bis 100°C erreicht werden.

Das aus dem Matrizenauslass austretende heiße Material wird mittels Messer kontinuierlich abgeschnitten und die so gebildeten Pellets werden in einem Kühler umgehend abgekühlt und ggf. nachgetrocknet. Der Rest-Feuchtigkeitsgehalt kann dabei im Bereich von etwa 1 bis15 Gew.-% bezogen auf das Pellet-Gesamtgewicht nach dem Kühlen und ggf Trocknen liegen.

In alternativer Weise kann das Pelletieren durch Extrusion oder Expansion (z.B. mittels Futter-Expandern der Firma Kahl, Deutschland). ersetzt werden (vgl z.B. "Feed Manufacturing Technology IV" Ed: mcEllhiney, Kansas State Univ.; Pub. by American Feed Industry Assoc, 1994; Arlington, VA. Darin: Extrusion Cooking Systems, Bob Hauck et al. S. 131-139; Wilson et al 1998, Journal of Poultry Science, 77 (Suppl.1): 41) Die Produkte aus diesen Prozessen können auch wieder gekrümelt werden.

### d2) Anwendung

Typische Futtermittelbestandteile die zur Herstellung erfindungsgemäßer Pellets brauchbar sind, umfassen Einzelfuttermitteln pflanzlichen oder tierischen Ursprungs gemäß FMV (Futtermittelverordnung), wie z.B. Getreidefolgeprodukte , Weizenfuttermehl, Weizenkleie; Extraktionsschrote, Trester, Melasseschnitzel, Fischmehl, Fleischknochenmehle; und/oder mineralischen Einzelfuttermitteln gemäß FMV , wie z.B. Carbonate, Phosphate, Sulfate, Propionate. Ebenfalls geeignet sind Getreide, wie Weizen , Roggen, Gerste, Hafer, Mais, Hirse oder Triticale; Getreidefolgeprodukte (Nebenprodukte der Müllerei), wie Kleien, Grießkleien, Weizengrießkleien, Futtermehle oder Nachmehle; Nebenprodukte aus der Ölgewinnung (Extraktionsschrote, Expeller, Kuchen); Nebenprodukte aus der Gewinnung von Zucker (Melasse, Trockenschnitzel, Futterzucker, Pülpe, Kartoffelstärke, Maiskleber, Weizenkleber); Nebenerzeugnisse des Gärungsgewerbes, Biertreber, Hefe, Malzkeime, Schlempe; sowie tierische und sonstige Futtermittel, wie Blutmehl, Fischmehl, Presssaft, Kartoffeleiweiß.

Insbesondere sind zu nennen Weizen, Körnermais, Gerste, Hafer; Sojabohnen; Getreidemehle, wie Weizen- oder Maismehl, Sojabohnenmehl, Rübenmelasseschnitzel, Weizengrießkleie, Weizennachmehl, Maiskleberfutter, Sojaextraktionsschrot, Rapsextraktionsschrot, Biertreber, Getreideschlempe, Rübenmelasse, Haferkleie; Zucker, wie Traubenzucker, und Zuckeralkohole, weiterhin proteinhaltige Bestandteile wie Sojakonzentrat, Fischmehl, Glutene wie Mais- oder Weizengluten, Öle und Fette, sowie Nutrazeutika , wie z.B. freie Aminosäuren, deren Salze, Vitamine (wie z.B. A, D, E) und Spurenelemente (wie z.B. Cu als CuSO₄), mineralische Bestandteile, wie Calciumcarbonat, Natriumchlorid; Phosphate, sowie gegebenenfalls Verarbeitungshilfsmittel, z. B. Gleitmittel, inerte Füllstoffe und dergleichen, und gegebenenfalls Konservierungsmittel.

Typische Milchleistungsfutterzusammensetzungen enthalten z. B. Mais, Weizen, Gerste, Hafer, Roggen, Zitrustrester, Sojaextraktionsschrot, Rapsextraktionsschrot, Sojaschalen, Palmkerexpeller, DDGS, Maiskleberfutter, Zuckerrübenschnitzel, Weizengrieskleie, Weizenkleie, Leinsaat, Melasse, Kalk, Salz, Vitamin- und Spurenelement-Vormischung.

Die Erfindung wird nun unter Bezugnahme auf folgende Ausführungsbeispiele näher erläutert.

### Experimenteller Teil

### Beispiel 1: Untersuchung der Milchfettverringerung bei Milchviehfütterung mit verschiedenen pelletierten CLA Futterformulierungen

### a) Pelletierung

Pelletpresse: Hersteller Simon Heessen, Type V3-30C.
Kapazität 600-650 kg/h
Matritze:
   Bohrungsdurchmesser: 5 mm
   Bohrungslänge 45 mm.
   Spaltbreite 1 mm
Pelletieremperatur: 80°C
Konditionierung : Wasserdampf T = 135 and 145°C (je nach Druck)

### b) Eingesetzte CLA Formulierungen

### (1) Lutalin ^{®}: (CLA -haltiges Handelsprodukt)

**Tabelle: Zusammensetzung von 5 verschiedenen Chargen**

| **Fettsäure** | | **1** | **2** | **3** | **4** | **5** |
|---|---|---|---|---|---|---|
| | | | | | | |
| Palmitinsäure | % ¹ | 4 | 4 | 5,3 | 6,4 | 6,5 |
| Stearinsäure | % | 3,6 | 3,6 | 3,6 | 3,5 | 3,6 |
| Oleinsäure | % | 28,4 | 28,4 | 26,5 | 23 | 22 |
| Linolsäure | % | 1,5 | 1,6 | 2,3 | 1,7 | 1,8 |
| CLA trans-10, cis-12, omega-6 | % | 29,9 | 29,9 | 29,6 | 30,4 | 31,0 |

| | | | | | | |
|---|---|---|---|---|---|---|
| ¹ GC Flächen-% | | | | | | |

| | | | | | | |
|---|---|---|---|---|---|---|
| CLA cis-9,trans-11 | % | 29,9 | 29,9 | 29,6 | 30,4 | 31,0 |
| CLA total | % | 59,8 | 59,8 | 59,2 | 60,8 | 62,0 |
| CLA trans, trans | % | 0,4 | 0,4 | 0,4 | 0,6 | 0,4 |
| Peroxidzahl, Ph.Eur. (B 057) | ppm | 17,9 | 6,3 | 7,1 | 13,5 | 11,9 |
| Säurezahl (ISO 660-96) | mg KOH/g | 14,0 | 14,1 | 14,0 | 14,9 | 12,9 |
| Wasser (ISO 4317) | % | 0,050 | 0,077 | 0,049 | 0,013 | 0,184 |
| Methanol (NF EN 14110) | ppm | 40,0 | 70,0 | 40,0 | 10,0 | 40,0 |
| | | | | | | |
| **Fettsäuren gesamt** | | **97,7** | **97,8** | **97,3** | **96,0** | **96,3** |

### (2) Lutrell ^{®}:

Lutrell besteht aus 34% Lutalin, sowie den Zusätzen 15% Silica, 5% Gips und 46% hydrierem Sojaöl, wobei vor allem letzteres die Pansen-stabilisierende Funktion erfüllt.

Die Zusammensetzung (Fettsäureprofil) von Lutrell ist in folgender Tabelle angegebene

| **Fettsäure** | **Anteil** | |
|---|---|---|
| | **(% Gesamtprodukt)** | |
| C14:0 | 0,12 % | |
| C15:0 | 0,00 % | |
| C16:0 | 7,94 % | |
| C16:1 | 0,04 % | |
| C17:0 | 0,00 % | |
| C18:0 | 41,05 % | |
| C18:1, c9 | 8,18 % | |
| C18:2, c9, c12 | 0,67 % | |
| C20:0 | 0,38 % | |
| C18:3 | 0,00 % | |
| CLA cis-9,trans-11 | 9,39 % | |
| CLA trans-10, cis-12, | 9,45 % | |
| C22:0 | 0,36 % | |
| C24:0 | 0,12 % | |
| Andere Fettsäuren | 2,42 % | |
| Andere Substanzen | 20,00 % | (anorganischer Träger) |
| | | |
| Σ | 100 % | |

### (3) Lutalin plus Silafett

Fettsäurezusammensetzung Silafett = gehärtetes (hydriertes) Sojaöl

| | |
|---|---|
| C12:0 | 0-0,5% |
| C14:0 | 0-1 % |
| C16:0 | 7-14% |
| C18:0 | 85-93% |
| C18:1 | 0-3% |
| C18:2 | 0-0.5% |
| Trans-Fettsäuren: | max. 1% |

### b) Versuchsschema

Es werden vier Versuchsvarianten (siehe folgende Tabelle) geprüft.
Variante 1 (T1) ist die Kontrolle (K) und enthält kein CLA,
Variante 2 (T2) enthält die CLA-Formulierung "Lutrell",
Variante 3 (T3) enthält die CLA-Formulierung "Lutalin" und
Variante 4 (T4) enthält die CLA-Formulierung "Lutalin plus Silafett".

| | **T1** | **T2** | **T3** | **T4** |
|---|---|---|---|---|
| Soja-Mais-Mix | 1000¹⁾ | 950 | 983,3 | 950,0 |
| Lutrell | 0 | 50 | 0,0 | 0,0 |
| Lutalin | 0 | | 16,7 | 16,7 |
| Lutalin plus Silafett | 0 | | 0,0 | 33,3 |
| | 1000 | 1000 | 1000 | 1000 |

### 1) Angaben in Gramm

Futter: 1 kg Ergänzer aus 25 % Sojaextraktionsschrot und 75 % Maisschrot, vorpelletiert

Die CLA-Präparate werden in einem Milchleistungsfutter (25 Gew.-% Sojaextraktionsschrot; 75 Gew.-% Maisschrot) eingemischt und pelletiert.

Das Versuchsschemaentspricht einem 4 x 4 lateinischen Quadrat (siehe folgende Tabelle) . Die reine CLA wird bei den Versuchsgruppen in gleichen Mengen von je 16,7g je Kuh und Tag dosiert

| | Gruppe 1 (n=5) | Gruppe 2 (n=5) | Gruppe 3 (n=5) | Gruppe 4 (n=5) |
|---|---|---|---|---|
| Periode 1 | K | Lutalin | Lutrell | Lutalin + Silafett |
| Periode 2 | Lutalin | K | Lutalin + Silafett | Lutrell |
| Periode 3 | Lutalin + Silafett | Lutrell | K | Lutalin |
| Periode 4 | Lutrell | Lutalin + Silafett | Lutalin | K |

Zuordnung zu Ergebnistabelle (Abschnitt f) unten): K= Kontrolle= T1, Lutrell = T2, Lutalin = T3 und Lutalin plus Silafett = T4.

Die Länge jeder Periode beträgt 21 Tage. D.h., nach einer Adaptationszeit von 14 Tagen erfolgt eine Messperiode von 7 Tagen. Alle vier Versuchsrationen basieren auf denselben Grundkomponenten.

### c) Tiere

Jede Behandlung wird nach obigem Versuchsschema mit 20 Kühen getestet. Weitere 4 Kühe werden als Reservetiere auf die Versuchsgruppen aufgeteilt. Die Versuchskühe befinden sich zu Versuchsbeginn in der mittleren Laktationsphase. Die mittlere Milchleistung liegt zu Versuchsbeginn bei ca. 32 kg FECM pro Tag. Die Versuchskühe (Deutsche Holstein) werden aus der Herde der Versuchsstation (Herdenleistung ca. 11 000kg Milch, 3,9% Fett, 3,4% Eiweiss) ausgewählt und nach den Kriterien
- Laktationsnummer (1 und höher)
- Milchfettgehalt (aufgrund der beiden vorangegangenen MLP's)
- Laktationstag und Milchleistung

in die vier Gruppen eingeteilt mit dem Ziel, vergleichbarer Gruppendurchschnittswerte einzustellen.

### d) Fütterung

Alle Kühe werden mit der gleichen Totalmischration (TMR) versorgt, die 1 Mal pro Tag zur *ad libitum* Aufnahme vorgelegt wird. Die TMR besteht aus Maissilage, Grassilage, Heu und ca. 45 % Kraftfutter. Die Zielwerte der TMR sind in nachstehender Tabelle dargestellt. Diese TMR stellt eine bedarfsdeckende Versorgung der Kühe für 33 kg Milchleistung sicher, wenn davon ca. 21 kg TM aufgenommen werden.

| | nXP g/kg T | XP g/kg T | XL g/kg T | XF g/kg T | NEL MJ/kg T |
|---|---|---|---|---|---|
| **Total** | **155** | **155** | **30** | **180** | **6,9** |

Gehalt der TMR an Rohnährstoffen gem. Weender Analyse (nXP=nutzbares Rohprotein, XP=Rohprotein, XL=Rohfett, XF=Rohfaser und NEL=Nettoenergie Laktion.

Von den drei CLA-Formulierungen werden zunächst pelletierte Vormischungen mit einem Milchleistungsfutter hergestellt. Diese Vormischungen werden dann in entsprechenden Anteilen (entsprechend 1 kg pro Tier und Tag) von Hand der TMR in den Futtertrögen zugemischt mit dem Ziel, eine Menge von jeweils 16,7 g der Reinsubstanzen in 20 kg TM der TMR einzustellen. Die Kontrollgruppe erhält das gleiche Kraftfutter in gleicher Menge ohne CLA.

### e) Probenahme und Messparameter

### Futter

Von der TMR wird täglich eine Probe zur Trockenmassebestimmung entnommen, die zu einer Sammelprobe pro Messperiode (7 Tage) vereinigt werden. Darin erfolgt die Bestimmung der Rohnährstoffgehalte nach der Weender Analyse und des Energiegehaltes nach dem Hohenheimer Futterwerttest.

### Kühe

Bei jeder Kuh wird täglich (individuell) die Aufnahme an TMR sowie die Milchleistung und die Lebendmasse erfasst. Während der 7-tägigen Messperiode werden drei Mal Milchproben als Aliquot aus Abend- und Morgengemelk genommen und auf die Gehalte an Fett, Gesamtprotein (Nx6,38), Lactose, Harnstoff und somatische Zellen durch den Milchprüfring Baden-Württemberg untersucht. In der Mitte der ersten und zweiten Woche jeder Versuchsperiode (Vorbereitungsphase) erfolgt eine zusätzliche Milchuntersuchung. Zusätzlich wird für jede Kuh und Behandlung eine die 7-tägige Messperiode umfassende Milch-Sammelprobe gebildet und eingefroren, um darin ggf. eine Fettsäurenanalyse des Milchfettes vornehmen zu können.

### f) Versuchsergebnisse

**Tabelle: Wirkung von Lutalin und Lutrell in pelletiertem Futter auf Milchfettverringerung bei Milchkühen, gefüttert mit 5 g trans-10/cis-12 CLA**

| **Treatment** | | **Milch kg** | **Fett kg** | **Protein kg** | **Protein %** | **Fett %** | **MFD** |
|---|---|---|---|---|---|---|---|
| | | | | | | | |
| T1 = Control | Mean | 34,4 | 1,310 | 1,153 | 3,40 | 3,86 | 0% |
| | SD | 6,56 | 0,232 | 0,170 | 0,38 | 0,58 | |
| T2 = Lutrell | Mean | 34,6 | 1,190 | 1,146 | 3,36 | 3,52 | - 9 % |
| | SD | 7,60 | 0,220 | 0,200 | 0,38 | 0,59 | |
| T3 = Lutalin | Mean | 35,1 | 1,218 | 1,156 | 3,34 | 3,52 | - 9 % |
| | SD | 7,27 | 0,218 | 0,183 | 0,37 | 0,54 | |
| T4 = Lutalin plus / Sillafett | | | | | | | - 9 % |
| | Mean | 34,7 | 1,205 | 1,151 | 3,36 | 3,50 | |
| | SD | 6,56 | 0,224 | 0,167 | 0,37 | 0,46 | |

| | | | | | | | |
|---|---|---|---|---|---|---|---|
| MEAN: Mittelwert SD: Standardabweichung | | | | | | | |

Überraschenderweise führten die Behandlungen T3 und T4 zur gleichen Milchfettreduktion wie die Behandlung T2. Dies obwohl der Wirkstoff CLA in T3 und T4 in einer nichtpansengeschützten Form in das Milchleistungsfutter eingemischt worden war. Das Pelletieren hat demnach den Wirkstoff CLA aus T3 und T4 im Pansen in identischem Umfang wie dies von Lutrell bekannt ist und auch durch T2 gezeigt wurde, geschützt.

### Beispiel 2: Untersuchung zur Pansenstbilität von verschiedenen pelletierten CLA Futterformulierungen

Die Stabilität im Pansen ist die wesentliche Voraussetzung für die Milchfettreduktion durch CLAhaltige Futtermittel. Hierzu wurden die unter Beispiel 1 getesteten Futtermittel (75% Mais plus 25% Sojaextraktionsschrot) mit 1,66 % Lutalin oder 5% Lutrell gemischt und pelletiert oder extrudiert. Die erzeugten Pellets und Extrudate wurden sodann in-vitro über 4, 12 und 24 Stunden inkubiert. Nach den vorgegebenen Zeiten wurde die Inkubation gestoppt, der Inhalt intoto in ein Gefäß übertragen und gefriergetrocknet. Aus diesem gefriergetrockneten Probenmaterial wurde anschließend der CLA-Gehalt bestimmt.

### a) Pelletierung und Extrusion

Zubereitung des Mehls: Mais und Sojaextraktionsschrot wurden zusammen eingewogen, gemahlen und in einem Horizontalmischer gemischt. Lutalin bzw. Lutrell wurden hinzugefügt und eingemischt. Die Mischzeit betrug 15 Minuten. Diese zwei Mischungen wurden jeweils geteilt. 80 kg wurden pelletiert und 30 kg wurden extrudiert.

Pelletmühle: Simon Heesen mit einer horizontalen Matrize, 3,3 x 35 mm, Nominalkapazität 300 kg/hr.

Extruder: Werner & Pfleiderer 37, Martrize 2 x Ø 3,9 mm, Nominalkapazität bis zu 50 kg/hr..

Pelletierung: Es wurden Pelletiertemperaturen von 67-68,5 ° C erreicht.

Extrusion: Es wurde eine Extrudiertemperatur von 85°C erreicht, ca. 14% Wasser waren zugefügt worden. Im Endprodukt war eine Restfeuchte von 10 bis 12% gefunden worden.

### b) Eingesetzte CLA Formulierungen

(1) Lutalin ^{®}: (CLA -haltiges Handelsprodukt. Zusammensetzung s. Beispiel 1.
(2) Lutrell ^{®} Pure: CLA-haltiges Handelsprodukt. Zusammensetzung s. Beispiel 1

### c) Versuchsschema

Es wurden 4 Versuchsvarianten (s. folgende Tabelle) geprüft.
Variante 1 (T1) Ein Pellet und enthält die CLA-Formulierung "Lutalin"
Varinate 2 (T2) Ein Pellet und enthält die CLA-Formulierung "Lutrell"
Variante 3 (T3) Ein Extrudat und enthällt die CLA-Formulierung "Lutalin"
Variante 4 (T4) Ein Extrudat und enthält die CLA-Formulierung "Lutrell"

| | **T1** | **T2** | **T3** | **T4** |
|---|---|---|---|---|
| Soja-Mais-Mix | 983,3* | 950 | 983,3 | 950,0 |
| Lutrell | | 50 | 0,0 | 50 |
| Lutalin | 16,7 | | 16,7 | 0 |
| Summe | 1000 | 1000 | 1000 | 1000 |

| | | | | |
|---|---|---|---|---|
| * Angaben in Gramm | | | | |

Futter: 1 kg Ergänzer aus 25% Sojaextraktionsschrot und 75% Maisschrot.

Eine unmittelbar an die Verarbeitung anschließende Analyse ergab, dass der Wirkstoff CLA die Pelletierung sehr gut überstand aber, dass die Extrusion zu ca. 40% Aktivitätsverlust führte.

### d) Inkubtion: Bestimmung von in-vitro Abbauprodukten beim Hohenheimer Futterwerttest (in vitro Test)

Der Hohenheimer Futterwert Test (HFT) ist ein in-vitro Verfahren zur Schätzung des energetischen Futterwertes von Wiederkäuerfuttermitteln. In einer angepassten Variante können mit dem HFT auch Wirkstoffe hinsichtlich ihrer Pansenbeständigkeit getestet werden, wenn die Inkubation nach definierten Zeiten gestoppt und der verbliebene Wirkstoff in der Inkubationslösung analysiert wird.

Die Durchführung des Hohenheimer Futterwerttests (HFT) erfolgt nach der Methodenvorschrift des VDLUFA (Methodenbuch Bd. III, Kap. 25.1).

Die Inkubationsdauer richtet sich nach der jeweiligen Fragestellung und liegt für den Abbau von CLA-Formulierungen zweckmäßiger weise zwischen 4 und 24 Stunden. Das in-vitro System ist bis 48 Stunden stabil, darüber hinaus ist jedoch mit Abweichungen von einem physiologischen Fermentationsverlauf zu rechnen. Von den Versuchsvarianten T1 bis T4 wurden jeweils 500 mg (entsprechend ca. 5 mg CLA bzw. ca. 3 mg für die extrudierten Varianten) eingewogen. Die Inkubationsdauer betrug 4, 12 und 24 h. Je Inkubationszeit wurde eine Trippelbestimmung (3 Inkubtionsgefäße je Versuchsvariante T1 bis T4) durchgeführt.

Nach den infrage kommenden Zeiten wurde die Inkubation durch Abkühlen in Eiswasser gestoppt. Das Pansensaft-Puffergemisch wurde jeweils quantitativ in Glasgefäße überführt, die zur anschließenden Gefriertrocknung geeignet sind. Die Trocknungsgefäße sind leer gewogen, so dass nach dem Trocknen das Restgewicht bestimmt und für die anschließende quantitative Analyse benutzt werden konnte. Als Gefriertrocknungsanlage wurde eine Gamma 1-20 der Fa. Christ, 37507 Osterode verwendet.

### e) Analysenmethode: Modifizierung der AM/00887/01

Aufgrund der besonderen Matrix und des niedrigen Gehalts an CLA mußte die in der Analysenmethode beschriebenen Aufarbeitung (siehe Kapitel 7.4.2) wie nachfolgend beschrieben modifiziert werden. Die vorgenommenen Änderungen sind in kursiver Schrift dargestellt.

Die Probe wird wie in 7.4.2 beschrieben in einen 250 ml Erlenmeyerkolben eingewogen bzw. vorgelegt, danach werden die angegebenen Mengen an BHT, Nartriumascorbat und Wasser zugesetzt.

Danach gibt man eine Spatelspitze des Enzyms Pronase hinzu und stellt den Erlenmeyerkolben für 15 Minuten in ein auf 60 °C temperiertes Ultraschallbad.

Nach Zugabe der in 7.4.2 beschriebenen Mengen an Ethanol und Essigsäure wird nochmals für 15 Minuten in das auf 60 °C temperierte Ultraschallbad gestellt.

Nach dem Abkühlen auf Raumtemperatur wird Extraktionslösung Cyclohexan/Ethylacetat 80:20 (v,v) zugesetzt. Anstelle von normalerweise 100 ml werden allerdings nur 50 ml der Extraktionslösung verwendet.

Danach wird wie in 7.4.2 beschrieben 30 Minuten auf dem Magnetrührer gerührt, man gibt 70 ml gesättigte Kochsalzlösung zu und rührt nochmals für 10 Minuten. Danach wird der Rührer abgestellt und die Phasentrennung abgewartet.

3 ml der organischen Phase werden entnommen, die Lösungsmittel Cyclohexan und Ethylacetat im Stickstoffstrom entfernt und der Rückstand in 2 ml der Extraktionslösung Cyclohexan/Ethylacetat 80:20 (v,v) aufgenommen (Aufkonzentrierung).

Ein Anteil der erhaltenen Probenlösung wird über einen 0,45 µm Einmalfilter direkt in ein HPLC-vial filtriert.

Das Injektionsvolumen beträgt 20 µl anstelle von normalerweise 10 µl.

### f) Versuchsergebnisse

### Retention (%) CLA-gesamt (als Methylester und als Frei Fettsäure)

| | **Einwaage, mg** | mg retention | | | Retention in % of starting weight | | | |
|---|---|---|---|---|---|---|---|---|
| | | Retention time, h | | | Retention time, h | | | |
| | **0** | **4** | **12** | **24** | 0 | **4** | **12** | **24** |
| Pellet Lutalin | 5,1 | 1,49 | 0,32 | 0,09 | 100% | 29% | 6% | 2% |
| Pellet Lutrell | 5,3 | 2,09 | 0,71 | 0,14 | 100% | **39%** | 13% | 3% |
| Extrudate Lutalin | 3,05 | 2,76 | 1,22 | 0,16 | 100% | 91% | **40%** | 5% |
| Extrudate Lutrell | 2,95 | 1,10 | 0,54 | 0,54 | 100% | 37% | 18% | 18% |

Die im Beispiel 1 bereits überraschenderweise festgestellte identische Milchfettreduktion von pelletiertem Lutalin gegenüber pelletiertem Lutrell konnte in diesem in-vitro Versuch insofern nachvollzogen werden, als die gemessenen Retentionen an CLA in beiden pelletierten Varianten T1 und T2 identisch waren. Somit können mit diesem Test zumindest solche Varianten sicher beurteilt werden, die auf dem gleichen Futter basieren und sich lediglich hinsichtlich einer physikalischen Behandlung unterscheiden. Überraschenderweise zeigten die extrudierten Varianten T3 nach 4 und 12 h und die T4 nach 12 und 24 h jeweils wesentlich höhere Retentionsraten als die pelletierten Varianten (vgl. Figur 1).

Somit kann die Schlussfolgerung gezogen werden, dass auch Lutalin (nicht Pansen-stabilisiertes CLA) in extrudierter Form eine mindestens gleichwertige Pansenstabilität wie ein vergleichbares Extrudat aus Lutrell (durch stabilisierende Zusätze Pansen-stabilisiertes CLA) hat.

## Patentansprüche

1. Pelletiertes Wiederkäuer-Futtermittel für Milchvieh, enthaltend in ungecoateter, pelletierter Form eine Mischung wenigstens eines festen partikelförmigen Futtermittelbestandteils mit wenigstens einem der Mischung zugesetzten Pansen-instabilen Inhaltsstoff, ausgewählt unter wenigstens eine konjugierten Linolsäuren (CLA),
wobei
der Anteil an zugesetztem Inhaltsstoff im Bereich von 0,1 bis 20 Gew.-%, bezogen auf das Gesamtgewicht der zu pelletierenden Mischung liegt und
das pelletierte Wiederkäuer-Futtermittel dadurch hergestellt wird, dass man
a. wenigstens einen festen partikelförmigen Futtermittelbestandteil mit dem Inhaltsstoff vermischt,
b. die so erhaltene Mischung gegebenenfalls durch Einleiten von bis zu 95°C heißem Wasserdampf konditioniert,
c. die gegebenenfalls konditionierte Mischung in einer Pelletier- Extrudier- oder Expandiervorrichtung unter Einwirkung von Druck und gegebenenfalls Dampf bei Temperaturen im Bereich von 60 bis 100°C zu Pellets verpresst, und
d. die so erhaltenen Pellets gegebenenfalls abkühlt und/oder nachtrocknet und gegebenenfalls anschließend krümelt.

2. Futtermittel nach Anspruch 1, wobei der Inhaltsstoff eine CLA ist, ausgewählt unter
a) cis/trans-9,11-Linolsäure;
b) cis/trans-8,10-Linolsäure;
c) cis/trans-11,13-Linolsäure;
d) cis/trans-10,12-Linolsäure; und
e) Gemischen von wenigstens zwei der oben genannten Verbindungen.

3. Pelletiertes Wiederkäuer-Futtermittel nach Anspruch 2,
wobei der wenigstens eine feste partikelförmige Futtermittelbestandteil ausgewählt ist unter Getreide,
Getreidefolgeprodukte, wie beispielsweise Nebenprodukte der Müllerei Nebenprodukte aus der Ölgewinnung, wie beispielsweise Extraktionsschrote, Expeller und Kuchen;
Nebenprodukte aus der Gewinnung von Zucker, wie beispielsweise Melasse, Trockenschnitzel, Futterzucker, Pülpe, Kartoffelstärke, Maiskleber und Weizenkleber;
Nebenerzeugnisse des Gärungsgewerbes, wie beispielsweise Biertreber, Hefe, Malzkeime und Schlempe; sowie
tierische und sonstige Futtermittel, wie beispielsweise Blutmehl, Fischmehl, Presssaft und Kartoffeleiweiß.

4. Futtermittel nach Anspruch 1 oder 3, wobei der Inhaltsstoff eine CLA ist, ausgewählt unter
a) 10-trans, 12-cis-Linolsäure; und
b) Gemischen von 9-cis,1 1-trans-Linolsäure und 10-trans, 12-cis-Linolsäure,

5. Futtermittel nach Anspruch 4, wobei die CLA ausgewählt ist unter Gemischen von 9-cis,11-trans-Linolsäure und 10-trans, 12-cis-Linolsäure.

6. Futtermittel nach Anspruch 5, wobei die CLA Mischung in Form einer der folgenden Fettsäurezusammensetzungen zugesetzt wird:
| **Fettsäure** | | **1** | **2** | **3** | **4** | **5** |
|---|---|---|---|---|---|---|
| | | | | | | |
| Palmitinsäure | % | 4 | 4 | 5,3 | 6,4 | 6,5 |
| Stearinsäure | % | 3,6 | 3,6 | 3,6 | 3,5 | 3,6 |
| Oleinsäure | % | 28,4 | 28,4 | 26,5 | 23 | 22 |
| Linolsäure | % | 1,5 | 1,6 | 2,3 | 1,7 | 1,8 |
| CLA trans-10, cis-12, omega-6 | % | 29,9 | 29,9 | 29,6 | 30,4 | 31,0 |
| CLA cis-9,trans-11 | % | 29,9 | 29,9 | 29,6 | 30,4 | 31,0 |
| CLA total | % | 59,8 | 59,8 | 59,2 | 60,8 | 62,0 |
| CLA trans, trans | % | 0,4 | 0,4 | 0,4 | 0,6 | 0,4 |
| Peroxidzahl, Ph.Eur. (B 057) | ppm | 17,9 | 6,3 | 7,1 | 13,5 | 11,9 |
| Säurezahl (ISO 660-96) | mg KOH/g | 14,0 | 14,1 | 14,0 | 14,9 | 12,9 |
| Wasser (ISO 4317) | % | 0,050 | 0,077 | 0,049 | 0,013 | 0,184 |
| Methanol (NF EN 14110) | ppm | 40,0 | 70,0 | 40,0 | 10,0 | 40,0 |
| | | | | | | |
| **Fettsäuren gesamt** | | **97,7** | **97,8** | **97,3** | **96,0** | **96,3** |
Wobei die %-Angaben Gaschromatographie-Flächen-% sind.

7. Verfahren zur Herstellung eines pelletierten Wiederkäuer-Futtermittels nach einem der vorhergehenden Ansprüche, wobei man
a. wenigstens einen festen partikelförmigen Futtermittelbestandteil mit wenigstens einem Inhaltsstoff gemäß der Definition in einem der Ansprüche 1 bis 6 vermischt,
b. die so erhaltene Mischung gegebenenfalls durch Einleiten von bis zu 95°C heißem Wasserdampf konditioniert
c. die gegebenenfalls konditionierte Mischung in einer Pelletier- Etrudier- oder Expandiervorrichtung unter Einwirkung von Druck und gegebenenfalls Dampf bei Temperaturen im Bereich von 60 bis 100°C zu Pellets verpresst; und
d. die so erhaltenen Pellets gegebenenfalls abkühlt und/oder nachtrocknet und gegebenenfalls anschließend krümelt.

8. Verfahren nach Anspruch 7 zur Herstellung eines Pansen-geschützten Wiederkäuer-Futtermittels, wobei man in Schritt a. eine Pansen-instabile Mischung des Inhaltsstoffs mit dem wenigstens einen Futtermittelbestandteil erhält
und
wobei die in Schritt b. erhaltene pelletierte Mischung im Vergleich zur nicht-pelletierten Mischung eine verbesserte Pansen-Stabilität des Inhaltsstoffs aufweist.

9. Futtermittel oder Verfahren nach einem der vorhergehenden Ansprüche, wobei man die zu pelletierende Mischung in einer als Ringmatrizenpresse ausgestaltete Pelletpresse pelletiert, indem man das vorkonditionierte Stoffgemisch mittels Walzen durch die Ringmatrize presst.

10. Futtermittel oder Verfahren nach einem der vorhergehenden Ansprüche, wobei die Ringmatrize einen Bohrungsdurchmesser von 4 bis 5mm und eine Lochlänge von 40 bis 50 mm aufweist.

11. Futtermittelnach einem der Ansprüche 1 bis 6, wobei der Anteil an zugesetztem Inhaltsstoff im Bereich von 1 bis 20 Gew.-%, insbesondere 3 bis 20 Gew.-%, bezogen auf das Gesamtgewicht der zu pelletierenden Mischung liegt.

## Claims

1. A pelleted ruminant feed for dairy cattle, comprising in uncoated, pelleted form a mixture of at least one solid particulate feed component with at least one rumen-unstable constituent which has been added to the mixture, selected from at least one conjugated linoleic acid (CLA),
wherein
the proportion of added constituent is in the range of from 0.1 to 20% by weight, based on the total weight of the mixture to be pelleted, and
the pelleted ruminant feed is prepared in that
a. at least one solid particulate feed component is mixed with the constituent,
b. the resulting mixture is optionally conditioned by introduction of steam at up to 95°C,
c. the optionally conditioned mixture is compressed in a pelleting, extruding or expanding device with the action of pressure and optionally steam at temperatures in the range of from 60 to 100°C to give pellets, and
d. the resulting pellets are optionally cooled and/or subjected to a final drying process and optionally subsequently crumbled.

2. The feed according to claim 1, wherein the constituent is a CLA, selected from among
a) cis/trans-9,11-linoleic acid;
b) cis/trans-8,10-linoleic acid;
c) cis/trans-11,13-linoleic acid;
d) cis/trans-10,12-linoleic acid; and
e) mixtures of at least two of the abovementioned compounds.

3. The pelleted ruminant feed according to claim 2,
wherein the at least one solid particulate feed component is selected from cereals,
cereal derivatives, such as by-products of milling;
by-products of oil production, such as extracted meals, expellers and cake;
by-products of the production of sugar, such as molasses, dry beet chips, feeding sugar, pulp, potato starch, maize gluten and wheat gluten;
by-products of the fermentation industry, such as brewer's grains, yeast, malt culms and stillage; and feedstuffs of animal and other origin, such as blood meal, fish meal, pressed must and potato protein.

4. The feed according to claim 1 or 3, wherein the constituent is a CLA, selected from among
a) 10-trans,12-cis-linoleic acid; and
b) mixtures of 9-cis,11-trans-linoleic acid and 10-trans,12-cis-linoleic acid.

5. The feed according to claim 4, wherein the CLA is selected from among mixtures of 9-cis,11-trans-linoleic acid and 10-trans,12-cis-linoleic acid.

6. The feed according to claim 5, wherein the CLA mixture is added in the form of one of the following fatty acid compositions:
| Fatty acid | | 1 | 2 | 3 | 4 | 5 |
|---|---|---|---|---|---|---|
| | | | | | | |
| Palmitic acid | % | 4 | 4 | 5.3 | 6.4 | 6.5 |
| Stearic acid | % | 3.6 | 3.6 | 3.6 | 3.5 | 3.6 |
| Oleic acid | % | 28.4 | 28.4 | 26.5 | 23 | 22 |
| Linoleic acid | % | 1.5 | 1.6 | 2.3 | 1.7 | 1.8 |
| CLA trans-10, cis-12, omega-6 | % | 29.9 | 29.9 | 29.6 | 30.4 | 31.0 |
| CLA cis-9,trans-11 | % | 29.9 | 29.9 | 29.6 | 30.4 | 31.0 |
| CLA total | % | 59.8 | 59.8 | 59.2 | 60.8 | 62.0 |
| CLA trans, trans | % | 0.4 | 0.4 | 0.4 | 0.6 | 0.4 |
| Peroxide number, Ph. Eur. (B 057) | ppm | 17.9 | 6.3 | 7.1 | 13.5 | 11.9 |
| Acid number (ISO 660-96) | mg KOH/g | 14.0 | 14.1 | 14.0 | 14.9 | 12.9 |
| Water (ISO 4317) | % | 0.050 | 0.077 | 0.049 | 0.013 | 0.184 |
| Methanol (NF EN 14110) | ppm | 40.0 | 70.0 | 40.0 | 10.0 | 40.0 |
| | | | | | | |
| Total fatty acids | | 97.7 | 97.8 | 97.3 | 96.0 | 96.3 |
where the % values are gas chromatography area% values.

7. A process for the preparation of a pelleted ruminant feed according to any of the preceding claims, wherein
a. at least one solid particulate feed component is mixed with at least one constituent as defined in any of claims 1 to 6,
b. the resulting mixture is optionally conditioned by introduction of steam at up to 95°C,
c. the optionally conditioned mixture is compressed in a pelleting, extruding or expanding device with the action of pressure and optionally steam at temperatures in the range of from 60 to 100°C to give pellets, and
d. the resulting pellets are optionally cooled and/or subjected to a final drying process and optionally subsequently crumbled.

8. The process according to claim 7 for the preparation of a rumen-protected ruminant feed, wherein in step a. a rumen-unstable mixture of the constituent with the at least one feed component is obtained
and
wherein the pelleted mixture obtained in step b. has an improved rumen stability of the constituent as compared to the unpelleted mixture.

9. The feed or process according to any of the preceding claims, wherein the mixture to be pelleted is pelleted in a pelleting press designed as an annular die press by pressing the pre-conditioned substance mixture through the annular die by means of rollers.

10. The feed or process according to any of the preceding claims, wherein the annular die has a bore diameter of 4 to 5 mm and a slot length of 40 to 50 mm.

11. The feed according to any of claims 1 to 6, wherein the proportion of added constituent is in the range of from 1 to 20% by weight, in particular 3 to 20% by weight, based on the total weight of the mixture to be pelleted.

## Revendications

1. Aliment pour animaux ruminants sous forme de granules destiné au bétail laitier, contenant, sous forme de granules non enrobés, un mélange d'au moins un constituant alimentaire pour animaux particulaire solide et d'au moins un ingrédient instable dans la panse, ajouté au mélange, choisi parmi au moins un acide linoléique conjugue (CLA),
dans lequel
la proportion d'ingrédient ajouté se situe dans la plage de 0,1 à 20% en poids, par rapport au poids total du mélange à transformer en granules et
l'aliment pour animaux ruminants sous forme de granules est préparé en ce qu'on
a. mélange au moins un constituant alimentaire pour animaux particulaire solide avec l'ingrédient,
b. conditionne le cas échéant le mélange ainsi obtenu par introduction de vapeur d'eau chaude jusqu'à 95°C,
c. presse le mélange le cas échéant conditionné dans un dispositif de granulation, d'extrusion ou d'expansion sous l'effet de pression et le cas échéant de vapeur à des températures dans la plage de 60 à 100°C en granules et
d. refroidit et/ou postsèche le cas échant les granules ainsi obtenus et ensuite les émiette le cas échéant.

2. Aliment pour animaux selon la revendication 1, l'ingrédient étant un CLA choisi parmi
a) l'acide cis/trans-9,11-linoléique ;
b) l'acide cis/trans-8,10-linoléique ;
c) l'acide cis/trans-11,13-linoléique ;
d) l'acide cis/trans-10,12-linoléique ; et
e) les mélanges d'au moins deux des composés susmentionnés.

3. Aliment pour animaux ruminants sous forme de granules selon la revendication 2,
ledit au moins un constituant alimentaire pour animaux particulaire solide étant choisi parmi les céréales,
les produits dérivés de céréales, comme par exemple les produits secondaires d'une meunerie,
les produits secondaires de la production d'huile, comme par exemple les tourteaux d'extraction, les tourteaux de pression et les tourtes ;
les produits secondaires de la production de sucre, comme par exemple la mélasse, la pulpe séchée, le sucre pour animaux, les pulpes, l'amidon de pomme de terre, le gluten de mais, le gluten de blé ;
les sous-produits de l'industrie de fermentation, comme par exemple les drêches, la levure, les germes de malt et la vinasse ; ainsi que
les aliments pour animaux d'origine animale et autres, comme par exemple la farine de sang, la farine de poisson, le jus pressé et les protéines de pomme de terre.

4. Aliment pour animaux selon la revendication 1 ou 3, l'ingrédient étant un CLA choisi parmi
a) l'acide 10-trans,12-cis-linoléique ; et
b) les mélanges d'acide 9-cis,11-trans-linoléique et d'acide 10-trans,12-cis-linoléique.

5. Aliment pour animaux selon la revendication 4, le CLA étant choisi parmi les mélanges d'acide 9-cis,11-trans-linoléique et d'acide 10-trans,12-cis-linoléique.

6. Aliment pour animaux selon la revendication 5, le mélange de CLA étant ajouté sous forme d'une des compositions d'acide gras suivantes :
| Acide gras | | 1 | 2 | 3 | 4 | 5 |
|---|---|---|---|---|---|---|
| | | | | | | |
| Acide palmitique | % | 4 | 4 | 5,3 | 6,4 | 6,5 |
| Acide stéarique | % | 3, 6 | 3, 6 | 3, 6 | 3,5 | 3, 6 |
| Acide oléique | % | 28,4 | 28,4 | 26,5 | 23 | 22 |
| Acide linoléique | % | 1,5 | 1, 6 | 2,3 | 1,7 | 1, 8 |
| CLA trans-10,cis-12,oméga-6 | % | 29,9 | 29,9 | 29, 6 | 30,4 | 31, 0 |
| CLA cis-9,trans-11 | % | 29,9 | 29,9 | 29, 6 | 30,4 | 31, 0 |
| CLA total | % | 59, 8 | 59, 8 | 59,2 | 60, 8 | 62, 0 |
| CLA trans, trans | % | 0,4 | 0,4 | 0,4 | 0, 6 | 0,4 |
| Indice de peroxyde, Ph.Eur. (B 057) | ppm | 17, 9 | 6,3 | 7, 1 | 13,5 | 11,9 |
| Indice d'acide (ISO 660-96) | mg de KOH/g | 14, 0 | 14, 1 | 14, 0 | 14, 9 | 12,9 |
| Eau (ISO 4317) | % | 0,050 | 0,077 | 0, 049 | 0,013 | 0,184 |
| Méthanol (NF EN 14110) | ppm | 40, 0 | 70, 0 | 40, 0 | 10, 0 | 40, 0 |
| | | | | | | |
| Acides gras, total | | 97,7 | 97, 8 | 97,3 | 96, 0 | 96,3 |
les indications en % étant des % surfaciques de la chromatographie en phase gazeuse.

7. Procédé de préparation d'un aliment pour animaux ruminants sous forme de granules selon l'une des revendications précédentes, dans lequel on
a. mélange au moins un constituant alimentaire pour animaux particulaire solide avec au moins un ingrédient selon la définition dans l'une des revendications 1 à 6,
b. conditionne le cas échéant le mélange ainsi obtenu par introduction de vapeur d'eau chaude jusqu'à 95°C,
c. presse le mélange le cas échéant conditionné dans un dispositif de granulation, d'extrusion ou d'expansion sous l'effet de pression et le cas échéant de vapeur à des températures dans la plage de 60 à 100°C en granules ; et
d. refroidit et/ou postsèche le cas échant les granules ainsi obtenus et ensuite les émiette le cas échéant.

8. Procédé selon la revendication 7 de préparation d'un aliment pour animaux ruminants protégé dans la panse, dans lequel on obtient, dans l'étape a., un mélange instable dans la panse de l'ingrédient et d'au moins un constituant alimentaire pour animaux
et
dans lequel le mélange sous forme de granules obtenu dans l'étape b. présente, par rapport à un mélange non sous forme de granules, une stabilité améliorée dans la panse de l'ingrédient.

9. Aliment pour animaux ou procédé selon l'une des revendications précédentes, le mélange à transformer en granules étant transformé en granules dans une presse de granulation équipée comme une presse à matrice annulaire, dans laquelle on presse le mélange de substances préconditionné au moyen de cylindres à travers la matrice annulaire.

10. Aliment pour animaux ou procédé selon l'une des revendications précédentes, la matrice annulaire présentant un diamètre de trou de 4 à 5 mm et une longueur d'orifice de 40 à 50 mm.

11. Aliment pour animaux selon l'une des revendications 1 à 6, la proportion d'ingrédient ajouté étant située dans la plage de 1 à 20% en poids, en particulier de 3 à 20% en poids, par rapport au mélange à transformer en granules.
